# EUROPEAN PATENT APPLICATION

(11) **EP 1 277 476 A1**
(43) Date of publication of application: **22.01.2003**
(21) Application number: 01921916.1
(22) Date of filing: 19.04.2001
(51) Int. Cl.: A61K 38/17, A61P 19/04, G01N 33/15

(54) **UTILIZATION OF ADAMTS-1 PROTEIN AND METHOD OF SCREENING AGGRECANASE ACTIVITY REGULATOR**

(30) Priority: 24.04.2000 JP 2000122650
(71) Applicant: Kureha Chemical Industry Co., Ltd., Tokyo 103-8552 (JP)
(72) Inventor: HIROSE, Kunitaka, Nerima-ku, Tokyo 179-0074 (JP); KUNO, Kouji, Kanazawa-shi, Ishikawa 920-0965 (JP); MATSUSHIMA, Kouji, Matsudo-shi, Chiba 271-0092 (JP)
(74) Representative: Minderop, Ralph H., Dr. rer. nat.
(86) International application number: JP0103357
(87) International publication number: WO01080876

(57) **Abstract**

New use of an ADAMTS-1 protein or a variation functionally equivalent thereto; a pharmaceutical composition for treating or preventing a disease caused by an abnormal re-modeling of cartilage, a pharmaceutical composition for normalizing an abnormal re-modeling of cartilage, and a pharmaceutical composition for decomposing an aggrecan, each comprising an ADAMTS-1 protein, or a variation functionally equivalent thereto, as an effective ingredient; and a method for screening an aggrecanase-activity-modulating agent useful for treating or preventing a disease caused by a depression or an elevation of an aggrecanase activity are disclosed.

The ADAMTS-1 protein, or a variation functionally equivalent thereto, can be used in the manufacture of a medicament to treat or prevent a disease caused by an abnormal re-modeling of cartilage, a medicament to normalize an abnormal re-modeling of cartilage, or a medicament to decompose an aggrecan.

Sequence Listing

<110> Kureha Chemical Industry Co., Ltd.

<120> Use of ADAMTS-1 protein, and method for screening agent for
modulating aggrecanase activity

<130> KRH-649

<140> JP 2000-122650
<141> 2000-04-24

<160> 2

<210> 1
<211> 33
<212> DNA
<213> Artificial Sequence

<220>
<223> Description of Artificial Sequence: an artificially synthesized
forward primer sequence

<400> 1
gtggcggccg cggctccaat gcagcgagct gtg                                33

<210> 2
<211> 32
<212> DNA
<213> Artificial Sequence

<220>
<223> Description of Artificial Sequence: an artificially synthesized
reverse primer sequence

<400> 2
cactctagac tccagccttc ttgctttccc tg                                32

## Description

### TECHNICAL FIELD

The present invention relates to a use of an ADAMTS-1 protein in the manufacture of a medicament to treat or prevent a disease caused by an abnormal re-modeling of cartilage, and a method for screening an agent for modulating an aggrecanase activity.

### BACKGROUND ART

An ADAMTS-1 (a disintegrin and metalloproteinase with thrombospondin type 1 motifs) protein was found as a first protein belonging to an ADAMTS family exhibiting a metalloproteinase activity as an ADAM type protein, and having thrombospondin type 1 motifs.

The ADAM family, that is, a group of proteins containing a disintegrin domain and a metalloproteinase domain, has a nucleotide sequence and an amino acid sequence which closely resemble those of the disintegrin and metalloproteinase of a snake venom (J. Cell Biol., 131, 275-278, 1995; Cell, 90, 589-592, 1997; Immunol. Today, 278, 278-284, 1999). When the ADAM family was found, it was thought that the disintegrin structure played an important role in an interaction of cells (Nature, 356, 248-252, 1992; Nature, 377, 652-656, 1995). However, subsequent investigations revealed that a cell-membrane binding metalloproteinase of the ADAM family exhibits an activity to cleave various proteins, such as a premature tumor necrosis factor a (TNF a) or a Drosophila delta protein, present on a cell surface, and release it from the cell surface (Nature, 385, 729-733, 1997; Nature, 385, 733-736, 1997; Science, 283, 91-94, 1999).

An ADAMTS-1 protein contains a reprolysin type matrix metalloproteinase (MMP) domain, and three thrombospondin type 1 motifs, and belongs to the ADAM family. An ADAMTS-1 gene was isolated from a colon cancer strain "colon 26" causing cancerous cachexia in vivo (J. Biol. Chem., 272, 556-562, 1997; Genomics, 46, 466-471, 1997). The TS type 1 motif of the ADAMTS-1 protein bonds to heparin, but the ADAMTS-1 protein is present while incorporated in an extracellular matrix (ECM) (J. Biol. Chem., 273, 13912-13917, 1998). It is thought that an MMP domain has a protease activity (J. Biol. Chem., 274, 18821-18826, 1999). Previous investigations by the inventors of the present invention revealed that the ADAMTS-1 protein is an extracellular matrix-binding type metalloproteinase, and it is suggested that the ADAMTS-1 protein bonds to a glycosaminoglycan sulfate in the extracellular matrix.

Further, it is known that an expression of the ADAMTS-1 gene is detected in human articular cartilage and an arthritic tissue (J. Biol. Chem., 274, 23443-23450, 1999; Biochem. Biophys. Res. Comm., 260, 318-322, 1999).

An aggrecan is a main proteoglycan present in an extracellular matrix of the articular cartilage, and a core protein thereof is modified by chondroitin sulfate and glycosaminoglycan keratan sulfate. The modification supplies water or imparts pliability to a cartilaginous tissue (FASEB J., 6, 861-870, 1992; Trend. Cell Biol., 5, 458-465, 1995).

The aggrecan is composed of plural domains, i.e., spherical domains (G1 and G2) at an N-terminus, glycosaminoglycan-binding region, and a spherical domain (G3) at a C-terminus (J. Biol. Chem., 262, 17757-17767, 1987). These aggrecans bond as monomers to hyaluronic acid via the G1 domains, and remain in the cartilage matrix in the form of an aggrecan aggregate.

It is known that, upon a cartilage destruction, a large amount of aggrecans are lost from the cartilaginous tissue because of a cleavage at two particular sites in the spherical domain of the aggrecan. The two positions are between the 341st aspartic acid residue and the 342nd phenylalanine residue (Asn341-Phe342), and the 373rd glutamic acid residue and the 374th alanine residue (Glu373-Ala374). An enzyme that cleaves the latter Glu373-Ala374 bond is called an aggrecanase (J. Biol. Chem., 266, 8683-8685, 1991; Biochim. J., 284, 589-593, 1992; J. Biol. Chem., 274, 23443-23450, 1999; J. Biochem., 126, 449-455, 1999).

Decomposed products obtained by the cleavage of the Glu373-Ala374 bond are detected in a cartilage or chondrocyte cultures (J. Biol. Chem., 266, 8683-8685, 1991; Biochim. J., 284, 589-593, 1992; Arch. Biochem. Biophys., 322, 22-30, 1995; J. Biol. Chem., 270, 2550-2556, 1995), or in synovial fluid of various articular diseases (J. Clin. Invst., 89, 1512-1516, 1992; Arthritis Rheum., 9, 1214-1222, 1993; J. Clin. Invst., 100, 93-106, 1997). As above, the aggrecanase plays an important role in the cartilage destruction of the arthrosis, and it is known that an elevation of the aggrecanase activity causes various articular diseases, such as rheumatoid arthritis, osteoarthritis, psoriatic arthritis, sclerosis, systemic lupus erythematosus, or articular damage. Further, it is suggested that an elevation of the aggrecanase activity causes tissue damage upon a cerebral infarction.

On the contrary, it is known that a depression of the aggrecanase activity causes an abnormalization of a cartilage re-modeling, that is, a destruction and regeneration of cartilage, and diseases caused by the abnormal re-modeling of cartilage are, for example, achondroplasia or hypertrophic osteoarthropathy.

Under the circumstances, the inventors of the present invention intensively investigated the functions of human ADAMTS-1 protein in a living body, and found that the ADAMTS-1 protein exhibits the aggrecanase activity.

The aggrecanase activity found by the present inventors for the ADAMTS-1 protein can be used for normalization of the abnormal re-modeling of cartilage, or a treatment or prevention of the diseases caused by the abnormal re-modeling of cartilage. Further, the ADAMTS-1 protein can be used to screen a compound capable of modulating (accelerating or inhibiting) the aggrecanase activity. Of the selected agents for modulating an aggrecanase activity, an agent for accelerating the aggrecanase activity is useful for treating or preventing a disease caused by a depression of the aggrecanase activity, for example, a disease caused by an abnormal re-modeling of cartilage. An agent for inhibiting the aggrecanase activity is useful for treating or preventing a disease caused by an elevation of the aggrecanase activity, for example, a disease caused by the cartilage destruction. The present invention is based on the above findings.

Accordingly, an object of the present invention is to provide a new use of the ADAMTS-1 protein; a pharmaceutical composition for treating or preventing a disease caused by an abnormal re-modeling of cartilage, a pharmaceutical composition for normalizing an abnormal re-modeling of cartilage, and a pharmaceutical composition for decomposing an aggrecan; and a method for screening an aggrecanase-activity-modulating agent useful for treating or preventing a disease caused by a depression or an elevation of an aggrecanase activity.

### DISCLOSURE OF INVENTION

The present invention relates to the use of an ADAMTS-1 protein, or a variation functionally equivalent thereto, in the manufacture of a medicament to treat or prevent a disease caused by an abnormal re-modeling of cartilage.

The present invention also relates to the use of an ADAMTS-1 protein, or a variation functionally equivalent thereto, in the manufacture of a medicament to normalize an abnormal re-modeling of cartilage.

Further, the present invention also relates to the use of an ADAMTS-1 protein or a variation functionally equivalent thereto in the manufacture of a medicament to decompose an aggrecan.

Further, the present invention also relates to a pharmaceutical composition for treating or preventing a disease caused by an abnormal re-modeling of cartilage, comprising an ADAMTS-1 protein or a variation functionally equivalent thereto, and a pharmaceutically acceptable carrier or diluent.

Further, the present invention also relates to a pharmaceutical composition for normalizing an abnormal re-modeling of cartilage, comprising an ADAMTS-1 protein or a variation functionally equivalent thereto, and a pharmaceutically acceptable carrier or diluent.

Further, the present invention also relates to a pharmaceutical composition for decomposing an aggrecan, comprising an ADAMTS-1 protein, or a variation functionally equivalent thereto, and a pharmaceutically acceptable carrier or diluent.

Further, the present invention also relates to a method for screening an agent for modulating an aggrecanase activity, comprising bringing an ADAMTS-1 protein of a variation functionally equivalent thereto into contact with an aggrecan, both in the presence and absence of a compound to be examined, and comparing a difference of changes in an aggrecan decomposition reaction.

The term "aggrecan" as used herein means a proteoglycan which is present in an extracellular matrix of an articular cartilage, and is composed of a core protein containing two spherical domains (G1 and G2) at an N-terminus, glycosaminoglycan-binding region, and a spherical domain (G3) at a C-terminus, and a chondroitin sulfate and a glycosaminoglycan keratan sulfate, each modifying the core protein.

Further, the term "aggrecanase activity" as used herein means an activity to decompose an aggrecan, for example, an activity to cleave a bond between the 373rd glutamic acid residue and the 374th alanine residue (Glu373-Ala374) in the core protein of human aggrecan.

### BRIEF DESCRIPTION OF DRAWINGS

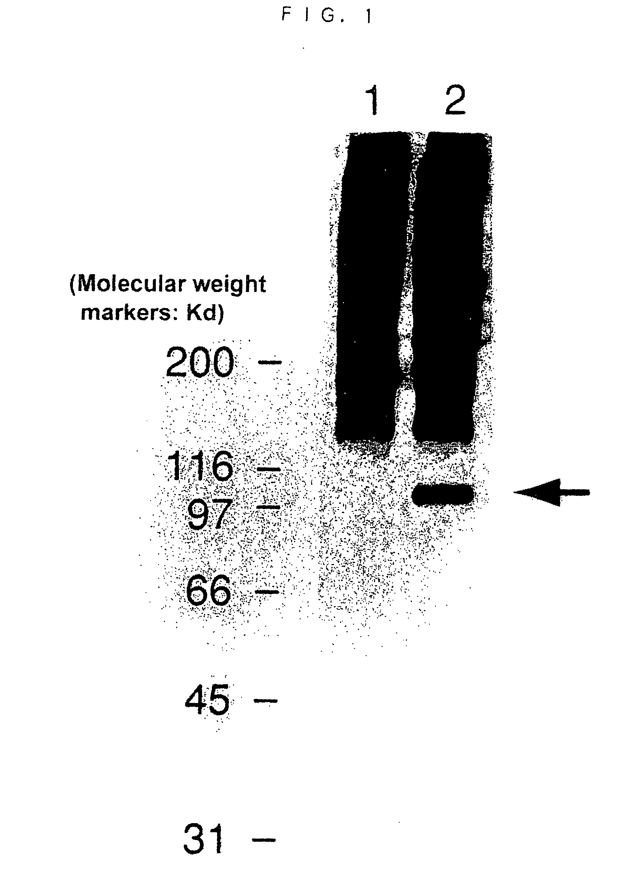

Figure 1 is a photograph showing the results of an electrophoresis of reaction products of a fraction containing a recombinant ADAMTS-1 protein or a control fraction with an aggrecan.

### BEST MODE FOR CARRYING OUT THE INVENTION

The present invention will be explained in detail hereinafter.

In the present invention, an ADAMTS-1 protein or a variation functionally equivalent thereto is used in (1) the manufacture of a medicament to treat or prevent a disease caused by an abnormal re-modeling of cartilage, (2) the manufacture of a medicament to normalize an abnormal re-modeling of cartilage, or (3) the manufacture of a medicament to decompose an aggrecan.

The term "ADAMTS-1 protein" as used herein means human ADAMTS-1 protein, and a protein corresponding to the human ADAMTS-1 protein in various animals, for example, mouse ADAMTS-1 protein (J. Biol. Chem., 272, 556-562, 1997). That is, the term "ADAMTS-1 protein" means a naturally occurring ADAMTS-1 protein in various animals including human. Human ADAMTS-1 protein consists of 727 amino acid residues, and an amino acid sequence thereof is disclosed in Japanese Unexamined Patent Publication No. 11-46781.

The term "variation functionally equivalent to an ADAMTS-1 protein" as used herein means a protein having an amino acid sequence wherein one or more (particularly one or several) amino acids are deleted in, substituted with, or inserted to the amino acid sequence of the naturally occurring ADAMTS-1 proteins, and exhibiting activities the same as those of the naturally occurring ADAMTS-1 proteins. Further, the term "activities the same as those of the ADAMTS-1 proteins" means activities to influence hemopoietic functions, for example, an activity to decrease the number of leukocytes and platelets, and at the same time, increase the number of erythrocytes, as disclosed in Japanese Unexamined Patent Publication No. 11-46781.

A form of the ADAMTS-1 protein, or a variation functionally equivalent thereto, which may be used in the present invention is not particularly limited, so long as the aggrecanase activity is maintained. For example, the naturally occurring ADAMTS-1 protein per se, or a fusion protein of the ADAMTS-1 protein or the variation functionally equivalent thereto with a fusion partner, such as a protein or peptide, may be used.

The fusion partner may be, for example, a protein or peptide for purification, such as glutathione S-transferase (GST) or hexapeptide of histidines, a protein or peptide for detection, such as β-galactosidase α peptide (LacZ), or a protein or peptide for expression, such as a signal sequence.

The ADAMTS-1 protein, or the variation functionally equivalent thereto, used in the present invention exhibits the aggrecanase activity, that is, an activity to cleave the aggrecan. Therefore, the ADAMTS-1 protein, or the variation functionally equivalent thereto, can be used not only in the manufacture of a medicament to decompose an aggrecan, but also in the manufacture of a medicament to normalize abnormalization derived from a depression of the aggrecanase activity, such as an abnormal re-modeling of cartilage, or in the manufacture of a medicament to treat or prevent a disease caused by the abnormalization, such as achondroplasia or hypertrophic osteoarthropathy.

(1) A pharmaceutical composition according to the present invention for treating or preventing a disease caused by an abnormal re-modeling of cartilage, (2) a pharmaceutical composition according to the present invention for normalizing an abnormal re-modeling of cartilage, and (3) a pharmaceutical composition according to the present invention for decomposing an aggrecan [hereinafter collectively sometimes referred to as a pharmaceutical composition according to the present invention] comprises the ADAMTS-1 protein or the variation functionally equivalent thereto as an effective ingredient, and a pharmaceutically or veterinarily acceptable carrier or diluent.

The formulation is not particularly limited to, but may be, for example, oral medicines, such as powders, fine subtilaes, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants; lubricants, flowability-enhancers, diluting agents, preservatives, coloring agents, perfumes, tasting agents, stabilizers, humectants, antiseptics, antioxidants, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

For the parenteral administration, for example, an injection such as a subcutaneous or intravenous injection, or the per rectum administration may be used. Of the parenteral formulations, an injection is preferably used.

When the injections are prepared, for example, watersoluble solvents, such as physiological saline or Ringer's solution, water-insoluble solvents, such as plant oil or fatty acid ester, agents for rendering isotonic, such as glucose or sodium chloride, solubilizing agents, stabilizing agents, antiseptics, suspending agents, or emulsifying agents may be optionally used, in addition to the ADAMTS-1 protein, or the variation functionally equivalent thereto, as the effective ingredient.

The pharmaceutical composition may be administered in the form of a sustained release preparation using sustained release polymers. For example, the pharmaceutical composition of the present invention may be incorporated to a pellet made of ethylenevinyl acetate polymers, and the pellet may be surgically implanted in a tissue for treatment or prevention.

The pharmaceutical composition of the present invention may contain the ADAMTS-1 protein, or the variation functionally equivalent thereto, in an amount of, but not particularly limited to, 0.01 to 99% by weight, preferably 0.1 to 80% by weight.

When the pharmaceutical composition of the present invention is utilized, the dose may be determined dependent upon the kind of disease, the age, sex, body weight, or symptoms of the subject, a method of administration, or the like. The pharmaceutical composition of the present invention may be orally or parenterally administered.

The pharmaceutical composition of the present invention may be used not only for the pharmaceutical application, but also for various applications, for example, in the form of eatable or drinkable products, such as functional foods or healthy foods, or feeds.

In the present method for screening an agent for modulating an aggrecanase activity, the ADAMTS-1 protein, or the variation functionally equivalent thereto, is used together with the aggrecan, a substrate of the aggrecanase. The ADAMTS-1 protein or the variation functionally equivalent thereto is brought into contact with the aggrecan, both in the presence and the absence of a compound to be examined, and a difference of changes in the aggrecan reactions under the conditions of the presence and the absence is compared. The "difference of the changes in the aggrecan reactions" may be, for example, an increase or decrease of products decomposed by the aggrecan.

The aggrecan which may be used in the screening method according to the present invention is not limited to human aggrecan, but may be aggrecans of mammals other than human, such as bovine, mouse or rabbit.

Further, the ADAMTS-1 protein which may be used in the screening method according to the present invention is not limited to human ADAMTS-1 protein, but may be ADAMTS-1 proteins of mammals other than human, such as bovine, mouse or rabbit.

In the screening method according to the present invention, a combination of the aggrecan and the ADAMTS-1 protein, or the variation functionally equivalent thereto, is not particularly limited. For example, the aggrecan and the ADAMTS-1 protein derived from the same species or the aggrecan and the ADAMTS-1 protein derived from the different species may be used.

The ADAMTS-1 protein or the variation functionally equivalent thereto cleaves the bond between the 373rd glutamic acid residue and the 374th alanine residue (Glu373 ―Ala374) in the core protein of the aggrecan, and therefore, polypeptides containing chondroitin sulfate and having about 100 kd are produced as the products decomposed by the aggrecan. As a means for comparing an amount of the aggrecan-decomposed products, a known assay for proteins, such as a Western blotting, may be used.

A step for bring the ADAMTS-1 protein, or the variation functionally equivalent thereto, into contact with the aggrecan is not particularly limited, so long as the step is carried out under the conditions that the aggrecanase activity of the ADAMTS-1 protein, or the variation functionally equivalent thereto, can be exhibited. For example, the step can be carried out in an aggrecanase reaction buffer as mentioned in Example 1(2).

When the amount of the products decomposed by the aggrecan in the presence of the compound to be examined is less than that of the products decomposed by the aggrecan in the absence of the compound to be examined, i.e., in a control test, the test compound can be judged as exhibiting an activity to inhibit the aggrecanase activity in the comparing step. The test compound having an activity to inhibit the aggrecanase activity, that is, an agent for inhibiting the aggrecanase activity, may be used as an effective ingredient in the pharmaceutical composition for treating or preventing a disease caused by the elevation of the aggrecanase activity, for example, a disease caused by a cartilage destruction, such as rheumatoid arthritis, osteoarthritis, psoriatic arthritis, sclerosis, systemic lupus erythematosus, or articular damage.

On the contrary, when the amount of the products decomposed by the aggrecan in the presence of the compound to be examined is more than that of the products decomposed by the aggrecan in the absence of the compound to be examined, i.e., in a control test, the test compound can be judged as exhibiting an activity to accelerate the aggrecanase activity. The test compound having an activity to accelerate the aggrecanase activity, that is, an agent for accelerating the aggrecanase activity, may be used as an effective ingredient in the pharmaceutical composition for treating or preventing a disease caused by the depression of the aggrecanase activity, for example, a disease caused by the abnormal re-modeling of cartilage, such as achondroplasia or hypertrophic osteoarthropathy.

The test compound which may be examined in the present method for screening the agent for modulating the aggrecanase activity is not particularly limited, but may be a naturally occurring compound or a synthesized compound. Further, a form of the test compound when examined in the present screening method is not particularly limited, but may be a purified form, or a non-purified crude form, such as a fermentation product, or a cell or tissue extract.

### EXAMPLES

The present invention now will be further illustrated by, but is by no means limited to, the following Examples.

### Example 1

### (1) Preparation of recombinant human ADAMTS-1 protein

A forward primer having a base sequence of SEQ ID NO.: 1:
gtggcggccgcggctccaatgcagcgagctgtg, a reverse primer having a base sequence of SEQ ID NO.: 2:
cactctagactccagccttcttgctttccctg,
and a human spleen cDNA library (Marathon-Ready cDNA; Clonetec Lab., Inc.; Palo Alto, CA, U.S.A.) were used to carry out a polymerase chain reaction (PCR) method in accordance with a conventional method, and isolate a gene (cDNA of about 3kbp) encoding a human ADAMTS-1 protein. The base sequence of the resulting cDNA was determined and found to be identical to the base sequence of the human ADAMTS-1 gene disclosed in Japanese Unexamined Patent Publication No. 11-46781.

Then, termini of the resulting cDNA were cleaved with a restriction enzyme XbaI and a restriction enzyme NotI, and the products were treated by an agarose gel electrophoresis. A DNA of about 3kbp was recovered from the gel, and ligated to an XbaI/NotI site of an animal cell expression vector pcDNA 3.1/V5-His (Invitrogen Corporation; Carlsbad, CA, U.S.A.) to constitute an ADAMTS-1 expression vector. A protein expressed by the ADAMTS-1 expression vector consists of a recombinant ADAMTS-1 protein having six histidine (His) residues incorporated at the C-terminus of the ADAMTS-1 protein (hereinafter referred to as merely "recombinant ADAMTS-1 protein").

The resulting ADAMTS-1 expression vector was incorporated into an animal cell strain HEK293, using a transfection reagent (lipofectamin; Life Technologies Inc.; Gaithersburg, Md., U.S.A.), in accordance with the protocol attached to the reagent. After 8 hours from the incorporation, the cells were washed with a serum-free Dulbecco modified Eagles' medium (DMEM) (Nissui; Tokyo, Japan) containing heparin (5 µg/mL), and then cultured for 2 days. Because the recombinant ADAMTS-1 protein contained six histidine (His) residues at the C-terminus, a nickel chelating column (Amersham Pharmacia Biotech; Uppsala, Sweden) was used to purify the recombinant ADAMTS-1 protein, using the protocol attached to the column, that is, a fraction containing a recombinant ADAMTS-1 protein was obtained.

The above culturing and purifying procedures were repeated except that animal cells to which the vector pcDNA3.1/V5-His without a gene encoding the recombinant ADAMTS-1 protein was incorporated were used instead of the animal cells to which the ADAMTS-1 expression vector was incorporated, whereby the control fraction corresponding to the recombinant ADAMTS-1 protein-containing fraction was obtained for use in an "Evaluation of aggrecanase activity" as mentioned below.

### (2) Evaluation of aggrecanase activity

A conventional method (Method of Hardingham; Biochem. J., 177, 237-247, 1979) was used to prepare aggrecan in use for evaluating an aggrecanase activity. More particularly, aggrecan was extracted from a bovine nasal cartilaginous tissue with 4 mol/L guanidine hydrochloride, and purified by cesium chloride density-gradient ultracentrifugation.

The recombinant ADAMTS-1 protein-containing fraction prepared in Example 1(1) in an amount of 0.5 µg as the amount of the recombinant ADAMTS-1 protein, or the control fraction was reacted with 10 µg of aggrecan in 100 µL of an aggrecanase reaction buffer [20 mmol/L tris-HCl (pH 7.6), 150 mmol/L NaCl, 5 mmol/L CaCl₂, 0.01% NaN₃] at 37°C for 12 hours.

After the reaction was completed, chondroitinase ABC (Seikagaku Corp.; Tokyo, Japan) (0.05 unit/10 µg of proteoglycan) and keratanase (Seikagaku Corp.) (0.05 unit/10 µg of proteoglycan) were added, and the whole was reacted in 200 µL of a sugar chain decomposition reaction solution [50 mmol/L tris-HCl (pH 8.0), 30 mmol/L sodium acetate, 10 mmol/L ethylenediaminetetraacetic acid (EDTA), 5 mmol/L phenylmethylsulfonyl fluoride (PMSF), 0.36 mmol/L pepstatin A, 10 mmol/L N-ethylmaleimide] at 37°C for 3 hours to decompose sugar chains.

The resulting product obtained by the above two reactions was treated in a Laemmli sample buffer [60 mmol/L tris-HCl (pH 6.8), 2% sodium dodecyl sulfate (SDS), 5% mercaptoethanol, 10% glycerol] at 100°C for five minutes, and then, a concentration-gradient polyacrylamide gel electrophoresis (gel concentration gradient = 2 to 15%) was carried out.

Proteins in the gel were transferred to a nitrocellulose membrane, according to a usual method, and then the membrane was treated in a blocking solution (Block ACE; Dainippon Pharmaceutical; Osaka, Japan), at 37°C overnight.

A Western blotting method was carried out, using an anti-chondroitin-4 sulfate antibody (2-B-6 antibody; recognizing and bonding to a core protein of aggrecan) as a first antibody, a horseradish peroxidase (HRP) binding sheep anti-mouse IgG polyclonal antibody (Amersham Pharmacia Biotech) as a second antibody, and a chemiluminescence system (ECL System; Amersham Pharmacia Biotech) as a detecting system.

The results of the Western blotting are shown in Fig. 1, where lane 1 shows the result of the control fraction, and lane 2 shows the result of the recombinant ADAMTS-1 protein-containing fraction. Further, in Fig. 1, lateral bars arranged in line from top to bottom at the left side of the lane 1 show positions of proteins used as molecular weight markers, and numerical figures located at the left sides of the lateral bars denote molecular weights (unit = kd) of the proteins used as the molecular weight markers.

A band as shown by an arrow at about 100 kd in the lane 2 was a core protein of aggrecan decomposed by the recombinant ADAMTS-1 protein. When EDTA, a chelating agent, was added in the reaction of the fractions and the aggrecan in the aggrecanase reaction buffer, a band did not appear at about 100 kd, although data was not shown. It is known that Ca ion is required for the expression of the aggrecanase activity. The above results show that the recombinant ADAMTS-1 protein has the aggrecanase activity.

### Preparation Example 1

The recombinant ADAMTS-1 protein-containing fraction prepared in Example 1(1) was dissolved in an amount of 25 mg/mL as an amount of the recombinant ADAMTS-1 protein in a physiological saline. The resulting recombinant ADAMTS-1 protein solution was transferred to an ampule, and the ampule was sealed to prepare the pharmaceutical composition of the present invention.

### INDUSTRIAL APPLICABILITY

According to the present invention, the ADAMTS-1 protein, or the variation functionally equivalent thereto, may be used in the manufacture of (1) a medicament to treat or prevent a disease caused by an abnormal re-modeling of cartilage, (2) a medicament to normalize an abnormal re-modeling of cartilage, or (3) a medicament to decompose an aggrecan.

Further, according to the present method for screening the agent for modulating the aggrecanase activity, the agent for accelerating the aggrecanase activity or the agent for inhibiting the aggrecanase activity can be selected. The agent for accelerating the aggrecanase activity may be used as an effective ingredient in the pharmaceutical composition for treating or preventing a disease caused by the abnormalization (for example, the abnormal re-modeling of cartilage) derived from the depression of the aggrecanase activity, such as achondroplasia or hypertrophic osteoarthropathy. Further, the agent for inhibiting the aggrecanase activity may be used as an effective ingredient in the pharmaceutical composition for treating or preventing a disease caused by the elevation of the aggrecanase activity, for example, a disease caused by a cartilage destruction, such as rheumatoid arthritis, osteoarthritis, psoriatic arthritis, sclerosis, systemic lupus erythematosus, or articular damage.

### Free text in Sequence Listing

A base sequence of SEQ ID NO.: 1 is a sequence of an artificially synthesized forward primer.

A base sequence of SEQ ID NO.: 2 is a sequence of an artificially synthesized reverse primer.

As above, the present invention is explained with reference to particular embodiments, but modifications and improvements obvious to those skilled in the art are included in the scope of the present invention.

## Claims

1. Use of an ADAMTS-1 protein, or a variation functionally equivalent thereto, in the manufacture of a medicament to treat or prevent a disease caused by an abnormal re-modeling of cartilage.

2. Use of an ADAMTS-1 protein, or a variation functionally equivalent thereto, in the manufacture of a medicament to normalize an abnormal re-modeling of cartilage.

3. Use of an ADAMTS-1 protein, or a variation functionally equivalent thereto, in the manufacture of a medicament to decompose an aggrecan.

4. A pharmaceutical composition for treating or preventing a disease caused by an abnormal re-modeling of cartilage, comprising an ADAMTS-1 protein, or a variation functionally equivalent thereto, and a pharmaceutically acceptable carrier or diluent.

5. A pharmaceutical composition for normalizing an abnormal re-modeling of cartilage, comprising an ADAMTS-1 protein, or a variation functionally equivalent thereto, and a pharmaceutically acceptable carrier or diluent.

6. A pharmaceutical composition for decomposing an aggrecan, comprising an ADAMTS-1 protein, or a variation functionally equivalent thereto, and a pharmaceutically acceptable carrier or diluent.

7. A method for screening an agent for modulating an aggrecanase activity, comprising bringing an ADAMTS-1 protein, or a variation functionally equivalent thereto, into contact with an aggrecan, both in the presence and absence of a compound to be examined, and comparing a difference of changes in an aggrecan decomposition reaction.
